# EUROPEAN PATENT SPECIFICATION

(11) **EP 3 769 733 B1**
(45) Date of publication and mention of the grant of the patent: **03.04.2024**
(21) Application number: 19771792.9
(22) Date of filing: 19.03.2019
(51) Int. Cl.: A61F 13/49, A61F 5/44

(54) **TAPE-TYPE DISPOSABLE DIAPER**
WEGWERFWINDEL VOM BANDTYP
COUCHE JETABLE DE TYPE À BANDE DE FIXATION

(30) Priority: 20.03.2018 JP 2018052977
(43) Date of publication of application: 27.01.2021
(73) Proprietor: Daio Paper Corporation, Ehime 799-0492 (JP)
(72) Inventor: OBATA, Masayo, Shikokuchuo-shi, Ehime 799-0431 (JP); MORITANI, Akie, Shikokuchuo-shi, Ehime 799-0431 (JP); MANABE, Sadanao, Tokyo 102-0071 (JP)
(74) Representative: Meissner Bolte Partnerschaft mbB
(86) International application number: PCT/JP2019/011370
(87) International publication number: WO 2019/181913

(56) References cited:
- EP-A1- 2 832 325
- JP-A- 2015 112 402
- JP-A- 2015 223 255
- JP-A- 2015 223 257
- JP-A- 2016 030 203
- JP-A- 2018 000 434
- US-A1- 2010 078 119

## Description

### Technical Field

The present invention relates to a tape-type disposable diaper having improved fitting around legs.

### Background Art

There are three main types of disposable diapers, a tape-type disposable diaper, an underpants-type disposable diaper, and a pad-type disposable diaper. Of these, a tape-type disposable diaper is worn by connecting portions provided on the left and right sides of a dorsal part to an outer surface of a ventral part after fitting the diaper to the body in a spread state

General tape-type disposable diapers have a crotch portion including the center in a front-back direction,a ventral part extending from the center in the front-back direction to the front side, and a dorsal part extending backward from the center in the front-back direction. At least the dorsal part has wing parts extending to the left and right of the crotch portion in the width direction. The ventral part and dorsal part have intermediate portions located between the left and right wing parts. The wing part has a connecting portion that is detachably connected to the outer surface of the ventral part. In use, the wing parts are turned from both sides of the waist to the outer surface of the ventral part to connect the connecting portions of the wing parts to the outer surface of the ventral part. Such tape-type disposable diapers are used not only for infants but also for nursing care (adults).

Conventionally, as a tape-type disposable diaper, a separate-type is known in which separately manufactured connecting tapes are attached to side edges of the main body forming the intermediate portion, and these connecting tapes become the wing parts (refer to, for example, Patent Literatures 4 and 5), in addition to a one unit-type in which side flap portions extending laterally from the side edges of an absorber are cut around the leg portions, and uncut portions are left as wing parts (refer to, for example, Patent Literatures 1 to 3). Further, as a separate type, it is also proposed to attach a unit including left and right wing parts to the body forming an intermediate portion (refer to, for example, Patent Literature 6).

Further types of disposable diapers are also known from Patent Literature 7 to 11.

However, in the conventional tape-type disposable diaper, when the wearer bends the thigh, the upper front surface of the thigh sometimes comes into tight contact with the lower edges of the wing parts of the ventral part. In particular, although this type of product typically accommodates a range of body sizes in one size, it has been found that when the wearer bends his/her thighs, even within the fitting range, as the upper limit is approached, the upper front of the thighs tends to come into tight contact with the lower edge of the wing parts of the ventral part.

### Citation List

### Patent Literature

Patent Literature 1: JP 2016-174816 A
Patent Literature 2: JP 2001-346830 A
Patent Literature 3: JP 2013-212213 A
Patent Literature 4: JP 2010-22550 A
Patent Literature 5: JP 2010-119463 A
Patent Literature 6: JP 2011-72736 A
Patent Literature 7: JP 2015-112402 A
Patent Literature 8: JP 2015-223255 A
Patent Literature 9: JP 2016-030203 A
Patent Literature 10: JP 2015-223257 A
Patent Literature 11: EP 2 832 325 A1

### Summary of Invention

### Technical Problem

Therefore, a main object of the present invention is to improve the fitting between the upper front surface of the thigh and the lower edge of the wing parts of the ventral part when the wearer bends the thigh.

### Solution to Problem

The various aspects of the tape-type disposable diaper which has solved the above-described problem are as follows.

### <First Aspect>

A tape-type disposable diaper including
a ventral part extending forward from the center in a front-back direction, a dorsal part extending backward from the center in the front-back direction, a crotch portion, both side edges of which extend from the middle in the front-back direction of the ventral part to the middle in the front-back direction of the dorsal part, both the side edges of the crotch portion passing through a width range of ± 5 mm away vertically from a direction intersecting with the front-back direction at a smaller angle less than ± 2 degrees,
an absorber incorporated in a range including the crotch portion,
wing parts extending outward in the width direction compared with the crotch portion in the ventral part and the dorsal part, and
a connecting tape for detachably connecting the ventral part both sides of the dorsal part.

In the tape-type disposable diaper,
a lower edge of the wing part of the ventral part extends so as to be located obliquely outward toward the front side,
the lower edge of the wing part of the ventral part has a first part located on the base edge side of the wing part and a second part located on the tip end side of the wing part,
the range excluding at least both ends of the first part and the range excluding at least both ends of the second part are each a wavy portion having a plurality of valleys recessed inward,
an obtuse side intersection angle between a tangent contacting both of adjacent valleys and the front-back direction in the wavy portion of the first part is 160 to 175 degrees,
an obtuse side intersection angle between a tangent contacting both of adjacent valleys and the front-back direction in a wavy portion of the second part is 120 to 150 degrees,
the dimension of the first part in the front-back direction is 0.15 to 0.30 times the dimension of the crotch portion in the front-back direction, and
the dimension of the second part in the front-back direction is 0.10 to 0.25 times the dimension of the crotch portion in the front-back direction.

### (Function and Effect)

In the ventral part of this tape-type disposable diaper, the lower edge of the wing part has the first part as a base part on the base edge side that is cut sufficiently deep toward the waist side, and the second part that bends laterally from the first part and extends sufficiently along the inguinal portion. Therefore, in this tape-type disposable diaper, even if the wearer greatly bends the thigh significantly, the first part is deeply cut up, such that the upper front surface of the thigh does not easily hit the lower edge of the wing part. Therefore, the tape-type disposable diaper is excellent in fitting between the upper front surface of the thigh and the lower edge of the wing part of the ventral part when the thigh is bent with a wider dimension around the leg.

Further, when a range excluding at least both ends of the first part and a range excluding at least both ends of the second part are wavy portions as in the present aspect, depending on the degree of bending deformation of projecting sections that bulges outward in the wavy portions, the positions of the lower edges of the wing parts can be changed. Then, since these projecting sections are deformed by contact with the body surface, the degree of bending deformation of the projecting sections is determined by the difference in body size and the degree of bending of the thigh. That is, the projecting sections are deformed according to the difference in body size, the degree of bending of the thigh, and the like, and fit the body surface. In particular, when the thigh is significantly bent, the projecting sections of the first part and the second part are bent, and the friction and contact between the thigh and the first part and the second part can be reduced. Moreover, rather than simply expanding spaces under the wing parts, the projecting sections cover the largest possible area. For this reason, it is easy to fit regardless of a difference in body size and bending of the thigh, and anxiety about leakage from around the leg can be reduced.

Note that, in the wavy portion, it does not matter whether the wave direction is linear or curved, and where the center line of the total amplitude (peak to peak) passes. In addition, the wavy portion also has both regular and irregular shapes, such as a shape that combines sinusoidal curves and arcs, a shape that these amplitudes and wavelengths change in the direction in which waves continue, and a shape that the bottom of the wave is sharp (bent) like a cloud shape. In addition, the first part and the second part at the lower edges of the wing parts of the ventral part, the side edges of the crotch portion, and the lower edges of the wing part on the dorsal part are edge portions that form the side edges of a single piece of a diaper. Regarding the front-back direction dimension of each edge, if both adjacent edges are wavy portions, the boundary between one portion and the other portion is an intersection of the lower edge and a bisector of the inner angle between a tangent that is in contact with both of adjacent valleys located on the most other side in one edge and y the tangent that is in contact with both the adjacent valleys located on the most one side at the other edge.

### <Second Aspect>

The tape-type disposable diaper according to the first aspect,
in which the wavy portion of the first part and the wavy portion of the second part each have a maximum total amplitude of 3 to 10 mm and a wavelength of 10 to 20 mm.

### (Function and Effect)

Usually, it is preferable that the size of the wavy portion be within the range of the present aspect. In particular, if the total amplitude of the wavy portion is excessively large, projecting sections may be folded between the surface of the diaper and the skin, which may lead to leakage and deterioration of wearing feeling.

### <Third Aspect>

The tape-type disposable diaper according to the first or second aspect, in which
both the side edges of the crotch portion extend straight or in a curved shape having no inflection point in a direction inclined obliquely inward by 1 to 2 degrees toward the front.

### (Function and Effect)

As in the present aspect, when the width of the crotch portion becomes narrower toward the front, the thighs are easily move forward. Therefore, when the thighs are bent, the fitting between the upper front surfaces of the thighs and the lower edges of the wing parts of the ventral part is improved. The crotch portion is susceptible to leg movements. For this reason, if both side edges of the crotch portion have wavy portions, the projecting sections may be folded between the surface of the diaper and the skin, which may lead to leakage and deterioration of wearing feeling. Therefore, it is preferable that both side edges of the crotch portion be linear, unlike the lower edges of the wing parts.

### <Fourth Aspect>

The tape-type disposable diaper according to any one of the first to third aspects, including,
side flap portions having no absorber on both sides in the width direction from the ventral part to the dorsal part,
a side elastic member provided on the width direction center side of the wing part of the ventral part in the side flap portions; and
a flat gather that contracts in a front-back direction by the side elastic member,
wherein a front-back direction range in which contraction force of the side elastic member acts has an overlapping range overlapping with at least a part of the front-back direction range of the first part overlap, and
the first part having a portion contracted by the side elastic member in a natural length state.

### (Function and Effect)

When flat gathers are provided as in the present aspect, a portion having the first part can also expand and contract to some extent in the front-back direction. Therefore, in a wide range of dimensions around legs, the fitting between the upper front surface of the thigh and the lower edges of the wing parts of the ventral part when the thighs are bent is improved.

### <Fifth Aspect>

The tape-type disposable diaper according to the fourth aspect,
in which the maximum elongation of the flat gather is 150 to 350%, and in the overlapping range, width direction intervals between the side elastic member and the first part is 15 to 50 mm at the maximum and 5 to 20 mm at the minimum.

### (Function and Effect)

When the distance between the first part and the side elastic member and the maximum elongation of the flat gather are within the range of the present aspect, it is preferable that the first part has a frill that is gently wavy in the thickness direction and has an excellent aesthetic appearance. In particular, when such a frill is combined with the shape of the first part, there is an advantage that the aesthetic appearance of the frill is further improved. On the other hand, if the distance between the first part and the side elastic member is excessively short, or if the maximum elongation of the flat gathers is excessively large, the finer frill waves not only reduce frill-likeness, but may also make the touch feeling harder. Further, if the distance between the first part and the side elastic member is excessively large, the projecting sections may be folded between the surface of the diaper and the skin, which may also lead to leakage and deterioration of wearing feeling. On the other hand, if the distance between the first part and the side elastic member is excessively large, or if the maximum elongation of the flat gathers is excessively short, frill formation may be insufficient. Note that, the maximum elongation means the maximum value of elongation in a stretchable direction (in other words, elongation in a spread state where the flat gathers are expanded flat without contraction or slack), and the length in a spread state is expressed as a percentage when the natural length is 100%.

### <Sixth Aspect>

The tape-type disposable diaper according to any one of the first to fifth aspects,
in which the lower edge of the wing part of the dorsal part extends so as to be located obliquely outward toward the back side from the back end of the side edge of the crotch portion,
at least a range excluding both ends in the lower edge of the wing part of the dorsal part is a wavy portion having a plurality of valleys recessed inward,
the wavy portion at the lower edge of the wing part of the dorsal part has an obtuse side intersection angle of 120 to 150 degrees formed by a tangent contacting both adjacent valleys and the front-back direction, and
the front-back dimension of the lower edge of the wing part of the dorsal part is 0.2 to 0.3 times the front-back dimension of the crotch portion.

### (Function and Effect)

Normally, the thigh has a small angle to bend backward. The wing part of the dorsal part preferably covers the gluteal region widely. Therefore, it is preferable that the lower edge of the wing part of the dorsal part greatly bend laterally from the back end of the crotch portion (asymmetrical in the front-back direction), unlike the wing part of the ventral part. Further, since the lower edge of the wing part of the dorsal part is also a wavy portion, there is the same advantage as the lower edge of the wing part of the ventral part. That is, also in the dorsal part, the position of the lower edge of the wing part can be changed according to the degree of bending deformation of the projecting sections (portions corresponding to the width of the waves). Then, since these projecting sections are deformed by contact with the body surface, the degree of bending deformation of the projecting sections is determined by the difference in body size and the degree of bending of the thigh. That is, the projecting sections are deformed according to the difference in body size, the degree of bending of the thigh, and the like, and fit the body surface. In particular, when the thigh is greatly bent, the projecting sections are bent, and the friction and contact between the lower edge of the wing part and the thigh can be reduced. Moreover, rather than simply expanding spaces under the wing parts, the projecting sections cover the largest possible area. For this reason, it is easy to fit regardless of a difference in body size and bending of the thigh, and anxiety about leakage from around the leg can be reduced.

### Advantageous Effects of Invention

According to the present invention, there is an advantage that, when the wearer bends the thigh, the fitting between the upper front surface of the thigh and the lower edge of the wing part of the ventral part is improved, and the like.

### Brief Description of Drawings

Fig. 1 is a plan view illustrating the inner surface of a tape-type disposable diaper in a state where a diaper is spread.
Fig. 2 is a plan view illustrating the outer surface of a tape-type disposable diaper in a state where a diaper is spread.
Fig. 3 is a cross-sectional view taken along line 6-6 in Fig. 1.
Fig. 4 is a cross-sectional view taken along line 7-7 in Fig. 1.
Fig. 5(a) is a cross-sectional view taken along line 8-8 in Fig. 1.
Fig. 5(b) is a cross-sectional view taken along line 9-9 in Fig. 1.
Fig. 6 is a cross-sectional view taken along line 5-5 in Fig. 1.
Fig. 7 is an enlarged plan view of an essential part of (a) a ventral part and (b) a dorsal part.
Fig. 8 is an enlarged plan view of an essential part of (a) a dorsal part and (b) a ventral part.
Fig. 9 is an enlarged plan view of a wavy portion.
Fig. 10 is a plan view illustrating the outer surface of a tape-type disposable diaper in a state where a diaper is spread.

### Description of Embodiments

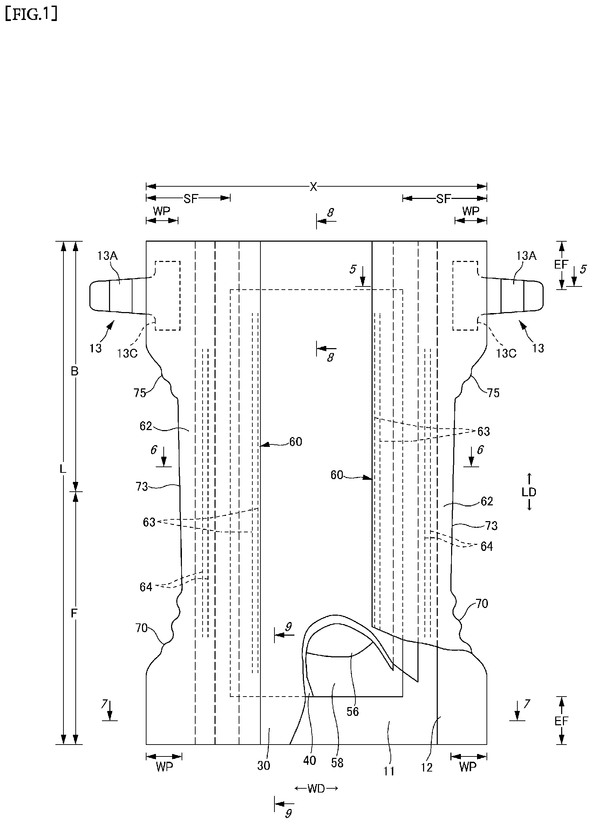

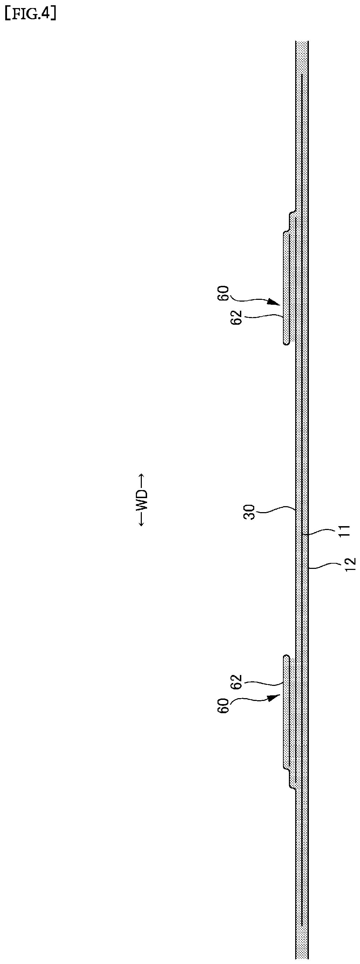

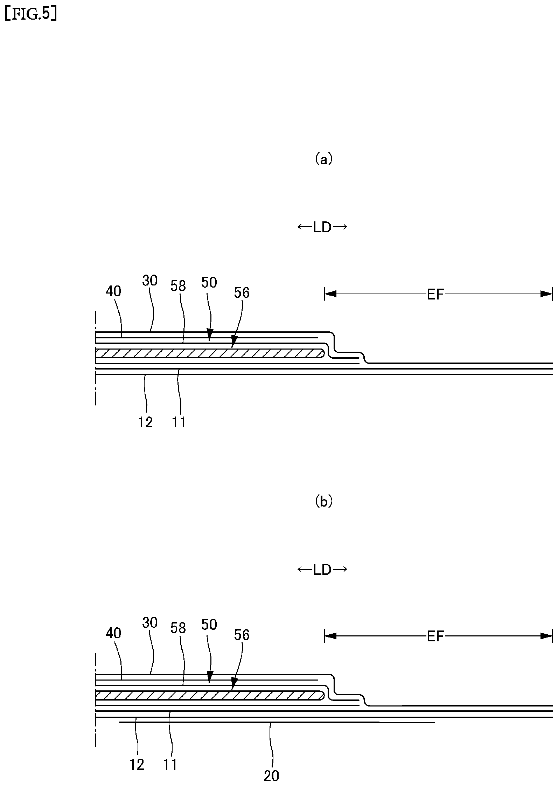

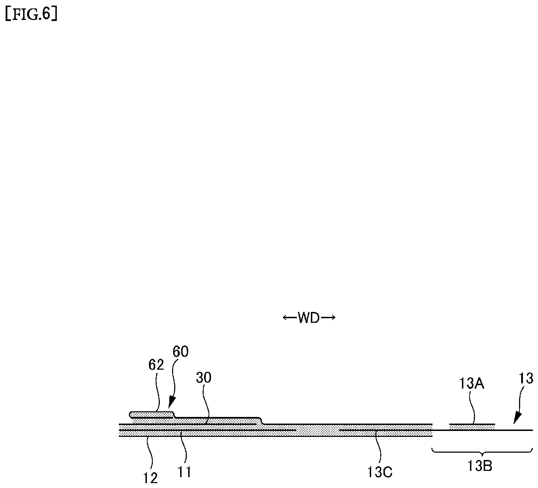

Figs. 1 to 6 illustrate examples of a tape-type disposable diaper, in which the reference character X indicates the maximum width of the diaper excluding a connecting tape, and the reference character L indicates the maximum length of the diaper. Dotted pattern portions in the drawings show a hot melt adhesive as a bonding means for bonding respective constituent members located on the front surface side and the back surface side thereof. The hot melt adhesive can be applied in a well-known method, such as slot coating, continuous linear or dotted linear bead coating, spiral spray coating, Z-shaped spray coating, or pattern coating (transfer of a hot melt adhesive in a letterpress method). In place of or in addition to this, in fixing portions of the elastic member, a hot melt adhesive can be applied to the outer peripheral surface of an elastic member to fix the elastic member to an adjacent member. Examples of the hot melt adhesive include, but are not limited to, adhesives of the EVA type, adhesive rubber type (elastomer-based), polyolefin-based, and polyester/polyamide-based. As a bonding means for bonding respective constituent members, a means by material welding such as heat sealing or ultrasonic sealing can also be used.

This tape-type disposable diaper has a ventral part F extending forward from the center in the front-back direction LD and a dorsal part B extending backward from the center in the front-back direction LD. Further, this tape-type disposable diaper includes an absorber 56 incorporated in a range including a crotch portion, a liquid pervious top sheet 30 covering the front surface side of the absorber 56, a liquid impervious sheet 11 covering the back surface side of the absorber 56, and an outer nonwoven fabric 12 that covers the back surface side of the liquid impervious sheet and constitutes the outer surface of the product.

The materials and characteristic parts of each portion will be described below in order.

### (Absorber)

The absorber 56 absorbs and retains excreted liquid, and can be formed of a fiber assembly. As this fiber assembly, besides those obtained by stacking short fibers such as fluff pulp and synthetic fibers, a filament assembly obtained by opening tows (fiber bundles) of synthetic fibers such as cellulose acetate as required can also be used. When fluff pulp or short fibers are accumulated, fiber basis weight can be set to, for example, about 100 to 300 g/m², and in the case of a filament assembly, fiber basis weight can be set to about 30 to 120 g/m². In the case of a synthetic fiber, the fineness is, for example, 1 to 16 dtex, preferably 1 to 10 dtex, more preferably 1 to 5 dtex. In the case of filament assembly, the filaments may be non-crimped fibers, but are preferably crimped fibers. The degree of crimp of the crimped fiber can be, for example, about 5 to 75 crimps, preferably 10 to 50 crimps, and more preferably about 15 to 50 crimps per 2.54 cm. In addition, crimped fibers which are uniformly crimped can be used.

### (Super Absorbent Polymer Particle)

The absorber 56 can contain super absorbent polymer particles partially or entirely. The super absorbent polymer particle means "powder" in addition to "particle". As the super absorbent polymer particles, those used for this type of absorbent articles can be used on an as-is basis. The particle diameter of the super absorbent polymer particles is not particularly limited, but for example, when the particles are sieved (shaking for five minutes) using a standard sieve (JIS Z8801-1:2006) of 500 um and the particles falling through the 500 um standard sieve are further sieved (shaking for five minutes) using the standard sieve (JIS Z8801-1: 2006) of 180 µm, desirably the proportion of the particles remaining on the 500 um standard sieve is 30% by weight or less, and the proportion of the particles remaining on the 180 um standard sieve is 60% by weight or more.

The material of the super absorbent polymer particles is not particularly limited, but materials having a water absorption capacity of 40 g/g or more are suitable. Examples of the super absorbent polymer particles include starch-based, cellulose-based, and synthetic polymer-based particles, and starch-polyacrylate acid (salt) graft copolymers, saponified starch-acrylonitrile copolymers, crosslinked sodium carboxymethylcellulose, and polyacrylate acid (salt) polymers can be used. As the shape of the super absorbent polymer particles, particulate materials which are usually used are preferable, but other shapes can also be used.

The super absorbent polymer particles having a water absorption rate of 70 seconds or less, particularly 40 seconds or less, are suitably used. If the water absorption rate is too slow, back-flow, in which the liquid fed into the absorber 56 returns to the outside of the absorber 56, is likely to occur.

As the super absorbent polymer particles, those having a gel strength of 1,000 Pa or more are preferably used. Thereby, even when the absorber 56 is bulky, it is possible to effectively suppress stickiness after liquid absorption.

The basis weight of the super absorbent polymer particles can be appropriately determined according to the absorption amount required for the use of the absorber 56. Therefore, although it cannot be said unconditionally, the basis weight can be 50 to 350 g/m². When the basis weight of the polymer is less than 50 g/m², it is difficult to ensure the absorption amount. If it exceeds 350 g/m², not only the effect is saturated, but also an excessive amount of super absorbent polymer particles causes a crunchy and uncomfortable feeling.

### (Package Sheet)

In order to prevent the super absorbent polymer particles from coming off or to improve the shape retention of the absorber 56, the absorber 56 can be incorporated as an absorbent element 50 wrapped with a package sheet 58. As the package sheet 58, a tissue paper, particularly a crepe paper, a nonwoven fabric, a polyethylene laminated nonwoven fabric, a sheet with small holes, and the like can be used. However, it is desirable that the sheet from which the super absorbent polymer particles do not come off is used. When a nonwoven fabric is used in place of a crepe paper, a hydrophilic SMMS (spun bond/melt blown/melt blown/spun bond) nonwoven fabric is particularly suitable, and polypropylene, polyethylene/polypropylene, and the like can be used as the material. The fiber basis weight is desirably 5 to 40 g/m², particularly desirably 10 to 30 g/m².

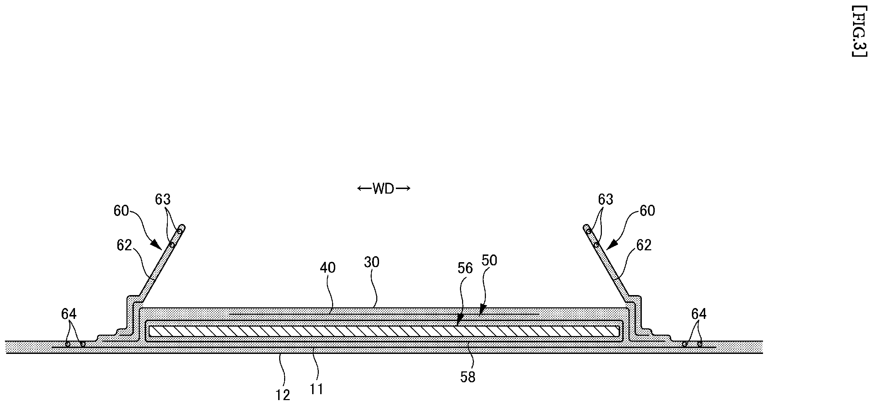

As illustrated in Fig. 3, the package sheet 58 has a structure in which the entire absorber 56 is wrapped with one sheet, or the package sheet 58 in which the entire absorber 56 may be wrapped with a plurality of sheets such as upper and lower two sheets. The package sheet 58 can be omitted.

### (Top Sheet)

The top sheet 30 has a property of permeating liquid, and for example, such as a perforated or nonporous nonwoven fabric and a porous plastic sheet can be used. Among them, a raw fiber of the nonwoven fabric is not particularly limited. Examples of the raw fiber include synthetic fibers such as polyolefin-based such as polyethylene and polypropylene, polyester-based, and polyamide-based, regenerated fibers such as rayon and cupra, natural fibers such as cotton, and mixed fibers and composite fibers in which two or more of these are used. Further, the nonwoven fabric may be manufactured by any processing. Examples of the processing method include known methods such as a spun lace method, a spun bond method, a thermal bond method, a meltblown method, a needle punch method, an air-through method, and a point bond method. For example, the spun lace method is preferable when flexibility and drapability are required, and the thermal bonding method is preferable when bulkiness and softness are required.

The top sheet 30 extends from the front end to the back end of the product in the front-back direction and extends laterally beyond the absorber 56 in the width direction WD. For example, such as when the starting point of the rising gathers 60, which will be described later, is located closer to the center in the width direction than the side edges of the absorber 56, if necessary, the top sheet 30 can be appropriately deformed such as making the width of the top sheet 30 shorter than the maximum width of the absorber 56.

### (Intermediate Sheet)

To quickly transfer liquid having permeated through the top sheet 30 to the absorber, it is possible to provide an intermediate sheet (also referred to as a "second sheet") 40 having a higher liquid permeation rate than the top sheet 30. The intermediate sheet 40 is intended to quickly transfer the liquid to the absorber to improve the absorption performance of the absorber and prevent the "returning" phenomenon of the absorbed liquid from the absorber. The intermediate sheet 40 can also be omitted.

Examples of the intermediate sheet 40 include the same material as the top sheet 30, a spun lace nonwoven fabric, a spunbond nonwoven fabric, SMS nonwoven fabric, a pulp nonwoven fabric, a mixed sheet of pulp and rayon, a point bond nonwoven fabric, or a crepe paper. In particular, an air-through nonwoven fabric is preferable because it is bulky. It is preferable to use a composite fiber having a core-sheath structure for the air-through nonwoven fabric. In this case, resin used for the core may be polypropylene (PP), but polyester (PET) having high rigidity is preferable. The basis weight is preferably 17 to 80 g/m², more preferably 25 to 60 g/m². The fineness of the raw fiber of the nonwoven fabric is preferably 2.0 to 10 dtex. To increase the bulkiness of the nonwoven fabric, it is also preferable to use eccentric fibers, hollow fibers, eccentric and hollow fibers, whose core is not in the center, as mixed fibers of all or a part of the raw material fibers.

Although the intermediate sheet 40 in the illustrated example is shorter than the width of the absorber 56 and disposed at the center, it may be provided throughout the maximum width. Further, the intermediate sheet 40 may be provided over the maximum length of the diaper, but may be provided only at the intermediate portion including the excretion position as in the illustrated example.

### (Liquid Impervious Sheet)

The liquid impervious sheet 11 is not particularly limited, but preferably has moisture permeability. As the liquid impervious sheet 11, for example, a microporous sheet can be preferably used which is obtained by stretching a sheet in one or two axial directions, the sheet being molded after kneading an inorganic filler in a polyolefin-based resin such as polyethylene or polypropylene. Further, as the liquid impervious sheet 11, a sheet which is made of a nonwoven fabric and has improved waterproofness can be used.

The liquid impervious sheet 11 desirably extends in the front-back direction LD and the width direction WD in the same or wider range as the absorber 56, but if there are other water blocking means, if necessary, it is also possible to adopt a structure in which the ends of the absorber 56 are not covered in the front-back direction LD and the width direction WD.

### (Outer Nonwoven Fabric)

The outer nonwoven fabric 12 covers the entire back surface side of the liquid impervious sheet 11 and makes the outer surface of the product look like a cloth. The outer nonwoven fabric 12 is not particularly limited. As a raw material fiber, for example, in addition to synthetic fibers such as polyolefin-based such as polyethylene or polypropylene, polyester-based, and polyamide type, regenerated fibers such as rayon and cupra, and natural fibers such as cotton can be used. As a processing method, a spun lace method, a spun bond method, a thermal bond method, an air-through method, a needle punch method, and the like can be used. However, a long-fiber nonwoven fabric such as a spunbonded nonwoven fabric, an SMS nonwoven fabric, and an SMMS nonwoven fabric are preferable in that both good touch feeling and strength can be compatible. In addition to using a single piece of nonwoven fabric, it is also possible to use multiple nonwoven fabrics in layers. In the latter case, it is preferable that the nonwoven fabrics are adhered to each other with a hot melt adhesive or the like. When a nonwoven fabric is used, the basis weight of the fiber is desirably 10 to 50 g/m², particularly desirably 15 to 30 g/m².

### (Rising Gather)

To prevent excrement that moves laterally on the top sheet 30 and to prevent side leakage, rising gathers 60 that stand up on the wearer's skin side are preferably provided on both sides of the surface in the width direction WD. It is obvious that the rising gather 60 can be omitted.

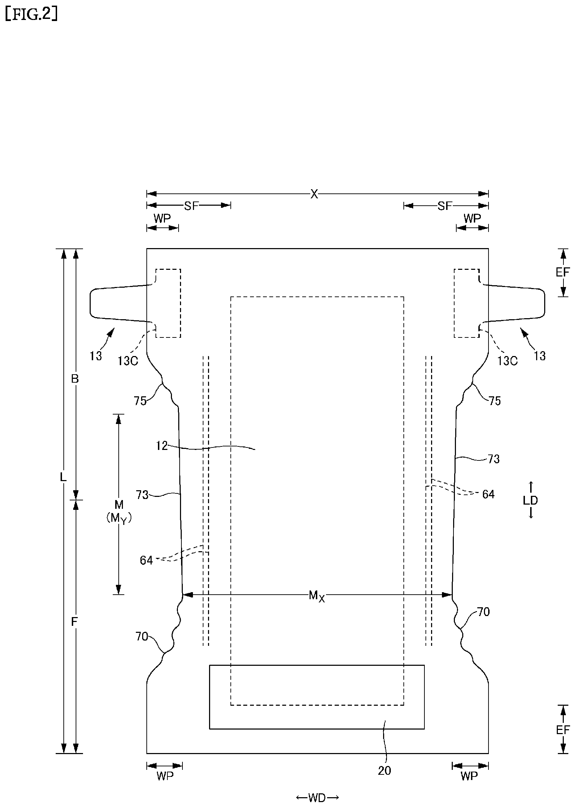

When the rising gather 60 is adopted, its structure is not particularly limited, and any known structure can be adopted. The rising gather 60 in the illustrated example is composed of a gather sheet 62 that is substantially continuous in the width direction WD, and an elongated gather elastic member 63 that is fixed to the gather sheet 62 in a stretched state along the front-back direction LD. As this gather sheet 62, a water repellent nonwoven fabric can be used, and rubber thread and the like can be used as the gather elastic member 63. As illustrated in Figs. 1 and 2, one or a plurality of the elastic member can be provided for each.

The inner surface of the gather sheet 62 has a joining start point in the width direction WD on the side portion of the top sheet 30, and the portion outside the joining start point in the width direction is the inner surface of each side flap portion SF, that is, in the illustrated example, the side portion of the liquid impervious sheet 11 and the side portion of the outer nonwoven fabric 12 located outside the liquid impervious sheet 11 in the width direction are bonded by a hot melt adhesive or the like.

In the periphery of the legs, the inside of the width direction from the joining start point of the rising gathers 60 is fixed on the top sheet 30 at both ends of the product front-back direction. However, the portion therebetween is a non-fixed free portion erected by contraction force of the elastic member 63 to close contact with the body surface.

### (End Flap Portion, Side Flap Portion)

The tape-type disposable diaper illustrated in the example includes a pair of end flap portions EF that do not have the absorber 56 and that extend to the front side and the back side of the absorber 56, respectively, and a pair of side flap portions SF that do not have the absorber 56 and that extend to the sides of both side edges of the absorber 56, respectively. The side flap portion SF may be made of a material (outer nonwoven fabric 12 or the like) continuous from the portion having the absorber 56 as in the illustrated example, or may be formed by attaching another material.

### (Flat Gather)

A side elastic member 64 made of an elongated elastic member such as a rubber thread is fixed to each side flap portion SF in a state of being extended along the front-back direction LD. As a result, the leg-around portion of each side flap portion SF is configured as a flat gather. The elastic members 64 around the leg portions is provided between the gather sheet 62 and the liquid impervious sheet 11 on the outer side in the width direction near the bonding start point of the bonded portion of the gather sheet 62 as in the illustrated example, and can also be provided between the liquid impervious sheet 11 and the outer nonwoven fabric 12 in the side flap portion SF. As in the illustrated example, a plurality of elastic members 64 around the leg portions may be provided on each side, or only one elastic member 64 may be provided on each side.

The flat gather is a portion where contraction force of the side elastic member 64 acts (in the drawing, a portion where the side elastic member 64 is illustrated). Therefore, in addition to a form in which the side elastic member 64 is provided only in the site of the flat gather, the following structure is included. In this structure, the side elastic members 64 extend to the front side, the back side, or both sides of the flat gather, and the side elastic member is finely cut at one place or a large number of places, or is not fixed to a sheet sandwiching the side elastic member 64, or both on the site other than the flat gather, whereby the contraction force of the side elastic member 64 does not act on the site other than the flat gather (substantially equivalent to not providing the elastic member), but the contraction force of the side elastic member 64 acts only on the flat gather site.

### (Connecting Tape)

The side flap portion SF of the dorsal part B is provided with a connecting tape 13 that is detachably connected to the outer surface of the ventral part F. When the diaper 10 is attached, the connecting tape 13 is turned from both sides of the waist to the outer surface of the ventral part F, and the connecting portion 13A of the connecting tape 13 is connected to an appropriate position on the outer surface of the ventral part F.

Although the structure of the connecting tape 13 is not particularly limited, in the illustrated example, a sheet base material forming a tape attachment part 13C fixed to the side flap portion SF and a tape main unit section 13B protruding from the tape attachment part 13C, and a connecting portion 13A with respect to the ventral side, which is provided at the intermediate portion in the width direction of the tape main unit section 13B in the seat base material. A tip end side of the connecting portion 13A is a tab part.

As the connecting portion 13A, a hook material (hook member) of a mechanical fastener (hook and loop fastener) may be provided, or an adhesive layer may be provided. The hook member has a number of engagement projections on its connecting surface, and the engagement projection has (A) a check mark shape, (B) a J shape, (C) a mushroom shape, (D) a T shape, and (E) a double J shape (a shape bonded back to back of a J shape), but may have any shape.

Further, as the sheet base material forming from the tape attachment part 13C to the tape main unit section 13B, nonwoven fabric, plastic film, poly-laminated nonwoven fabric, paper or a composite material of these can be used, but a spunbond nonwoven fabric, an air-through nonwoven fabric, or a spunlace nonwoven fabric, having a fineness of 1.0 to 3.5 dtex, a basis weight of 20 to 100 g/m², a thickness of 1 mm or less is preferable.

### (Target Sheet)

It is preferable to provide a target portion at a connecting portion of the connecting tape 13 in the ventral part F. The target portion can be provided by attaching a target sheet 20 for facilitating the connection to the outer surface of the ventral part F as in the illustrated example. In the case where the connecting portion 13A is the hook member, the target sheet 20 can be used in which a large number of loop threads to which an engagement projection of a hook member is tangled are provided on a surface of the sheet base member made of a plastic film or a nonwoven fabric. Further, in the case of an adhesive layer, it is possible to use a sheet base material made of a plastic film having a smooth surface with high adhesiveness and subjected to peel treatment. Further, when the connecting portion of the connecting tape 13 in the ventral part F is made of a nonwoven fabric, for example, when the outer nonwoven fabric 12 is provided as in the illustrated example, the target sheet 20 is omitted and the hook material is entangled and connected as the fiber of the outer nonwoven fabric 12. In this case, the target sheet 20 as a mark may be provided between the outer nonwoven fabric 12 and the liquid impervious sheet 11, or the mark may be printed on the outer surface of the outer nonwoven fabric 12 or the liquid impervious sheet 11.

### (Wing Part)

This tape-type disposable diaper, as illustrated in Figs. 1, 2, and 10, has a crotch portion M extending from the middle in the front-back direction LD of the ventral part F to the middle in the front-back direction LD of the dorsal part B. Both side edges of the crotch portion M pass a width range of ± 5 mm away vertically from a direction intersecting with the front-back direction LD at an angle *θ* ₀ less than ± 2 degrees. Further, the ventral part F and the dorsal part B have a wing part WP that extends outward of the crotch portion M in the width direction WD. The lower edge 70 of the wing part of the ventral part F extends so as to be located obliquely outward from the front end of the side edge of the crotch portion M toward the front, and the lower edge 75 of the wing part of the dorsal part B extends so as to be positioned obliquely outward toward the back side from the back end of the side edge of the crotch portion M. The side edge of the wing part WP has a linear shape in the illustrated example, but is not limited thereto, and another known shape can be adopted. The dimension of the crotch portion M in the front-back direction LD can be appropriately determined, but can be set to about 1.2 to 1.4 times the minimum width M_{X} of the crotch portion M. For infants, the dimension M_{Y} of the crotch portion M in the front-back direction LD is about 10 to 30 cm.

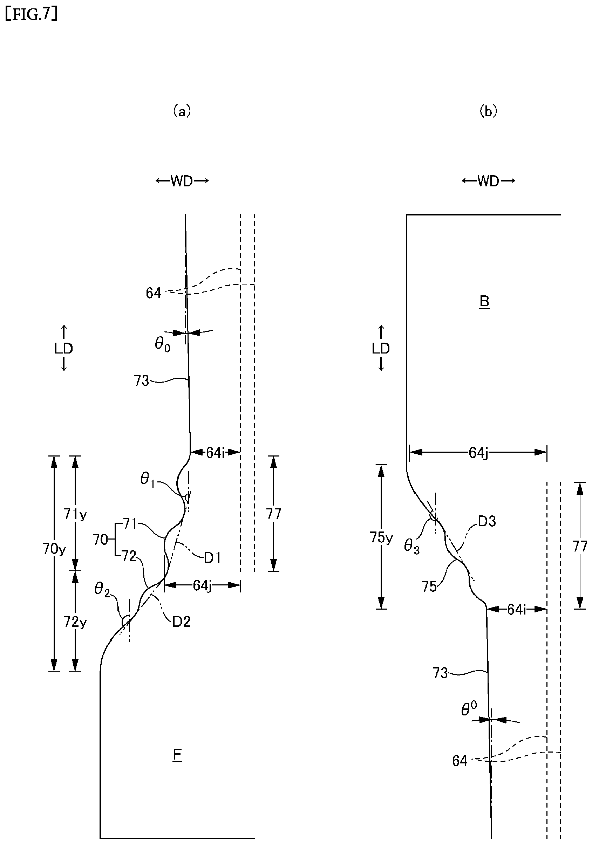

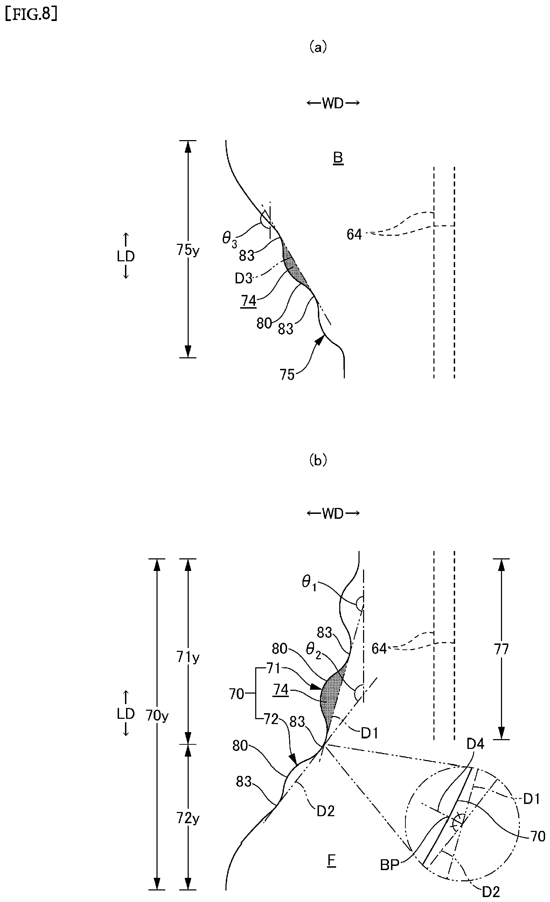

As illustrated in Figs. 7(a) and 8(b) in an enlarged manner, the lower edge 70 of the wing part WP of the ventral part F has a first part 71 located on the base edge side of the wing part WP, and a second part 72 located on the tip end side of the wing part WP. The range excluding at least both ends of the first part 71 (preferably the whole as in the illustrated example) and the range excluding at least both ends of the second part 72 (preferably the whole as in the illustrated example) are the wavy portion 80 having a plurality of valleys recessed inward. In the wavy portion 80 of the first part 71, the obtuse side intersection angle θ₁ between the tangent D1 that contacts both of the adjacent valleys 83 and the front-back direction LD is 160 to 175 degrees (preferably 163 to 170 degrees). In the wavy portion 80 of the second part 72, the obtuse side intersection angle θ₂ between the tangent D2 contacting both of the adjacent valleys 83 and the front-back direction LD is 120 to 150 degrees (preferably 130 to 140 degrees).

Further, the dimension 71y of the first part 71 in the front-back direction LD is 0.15 to 0.30 times (preferably 0.20 to 0.25 times) the dimension M_{Y} in the front-back direction LD of the crotch portion M. On the other hand, the dimension 72y of the second part 72 in the front-back direction LD is 0.10 to 0.25 times (preferably 0.15 to 0.20 times) the dimension M_{Y} in the front-back direction LD of the crotch portion M. The dimension 71y of the first part 71 in the front-back direction LD and the dimension 72y of the second part 72 in the front-back direction LD add up to the dimension 70y.

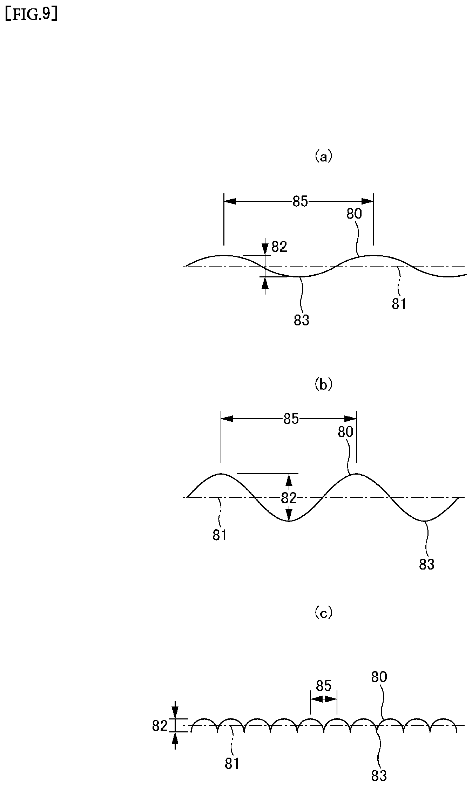

Here, as described above, in the wavy portion 80, it does not matter whether the wave direction is linear or curved, and where the center line of the total amplitude (peak to peak) passes. It is obvious that the wavy portion 80 includes, as in the example illustrated in Fig. 9, a portion that continues with a constant amplitude 82 (± 5 mm) around a specific direction 81. Further, the wavy portion 80 includes both regular and irregular shapes, such as a part that combines sinusoidal curves (for example, the wavy portion 80 illustrated in Fig. 9(a)) and arcs (for example, the wavy portion 80 illustrated in Fig. 9(b)), a part where these amplitudes and wavelengths change in the direction in which the waves continue, and a part where the bottom of the wave is sharp (bent) like a cloud shape (for example, the wavy portion 80 illustrated in Fig. 9(c)). From the viewpoint of improving the touch, the wavy portion 80 preferably has an outwardly bulging part having a rounded shape such as an arc. The wavy portion 80 in the example illustrated in Figs. 7 and 8 is formed by alternately connecting arcs, and in this case, the radius of the arcs is preferably about 5 to 15 mm.

The waves of the wavy portion 80 of the first part 71 and the wavy portion 80 of the second part 72 may be continuous or discontinuous. When the wavy portion 80 of the first part 71 and the wavy portion 80 of the second part 72 are interrupted, a bent portion where the direction changes from the first part 71 to the second part 72 and both ends of the lower edge 70 of the wing part WP of the ventral part F may have a shape in which corners are cut as in the illustrated example or a shape in which corners are firmly left. In the former case, the corners are preferably rounded, and particularly preferably the corners are arcuate with a radius of about 20 to 80 mm.

The first part 71 and the second part 72 at the lower edge 70 of the wing part WP of the ventral part F, the side edge of the crotch portion M, and the lower edge 75 of the wing part WP on the dorsal part B are an edge portion that forms the side edge of a single piece of a diaper. Regarding the front-back direction LD dimension of each edge, if both adjacent edges are a wavy portion 80, the boundary between one part and the other part is the intersection of the lower edge 70 and the bisector of the inner angle between a tangent that is in contact with both of the adjacent valleys 83 located on the most other side in one edge and the tangent that is in contact with both the adjacent valleys 83 located on the most one side at the other edge. For example, as illustrated in Fig. 8, the boundary between the first part 71 and the second part 72 is an intersection point BP of the lower edge 70 and a bisector D4 of the inner angle between the tangent D1 which is in contact with both of the adjacent valleys 83 located on the most front side in the first part 71 and the tangent D2 which is in contact with both of adjacent valleys 83 located on the most back side in the second part 72.

In the ventral part F of this tape-type disposable diaper, the lower edges 70 of the wing part has the first part 71 as a base part on the base edge side that is cut sufficiently deep toward the waist side, and the second part 72 that ends laterally from the first part 71 and extends sufficiently along the inguinal portion. Therefore, in the present tape-type disposable diaper, even if the wearer bends the thigh significantly, the first part 71 is deeply cut up, such that the upper front surface of the thigh is not easily tightly attached to the lower edge 70 of the wing part. Therefore, in this tape-type disposable diaper, the fitting between the upper front surface of the thigh and the lower edge 70 of the wing part of the ventral part F is improved when the thigh is bent in wide dimensions around legs. Further, when the range excluding at least both ends of the first part 71 and the range excluding at least both ends of the second part 72 are wavy portions 80, depending on the degree of bending deformation of the projecting section 74 (a part indicated by a dotted pattern in Fig. 8) that bulges outward in the wavy portion, the position of the lower edge 70 of the wing part WP can be changed. Then, since this projecting section 74 is deformed by contact with the body surface, the degree of bending deformation of the projecting section 74 is determined by the difference in body size and the degree of bending of the thigh. That is, the projecting section 74 is deformed according to the difference in body size, the degree of bending of the thigh, and the like, and fits the body surface. In particular, when the thigh is greatly bent, the projecting sections 74 of the first part 71 and the second part 72 are bent, and the friction and contact between the thigh portion and the first part 71 and the second part 72 can be reduced. Moreover, rather than simply expanding the space under the wing parts WP, the projecting section 74 covers the largest possible area. For this reason, it is easy to fit regardless of a difference in body size and bending of the thigh, and anxiety about leakage from around the leg can be reduced. From these viewpoints, the first part 71 preferably has about two to five projecting sections 74. In addition, the second part 72 preferably has about two to five projecting sections 74.

The dimensions of the wavy portion 80 of the first part 71 and the wavy portion 80 of the second part 72 can be appropriately determined, but in the normal case, it is preferable that the maximum value of the total amplitude 82 be 3 to 10 mm, and the wavelength 85 be 10 to 20 mm. In particular, if the total amplitude 82 of the wavy portion 80 is excessively large, the projecting sections 74 may be folded between the surface of the diaper and the skin, which may lead to leakage and deterioration of wearing feeling.

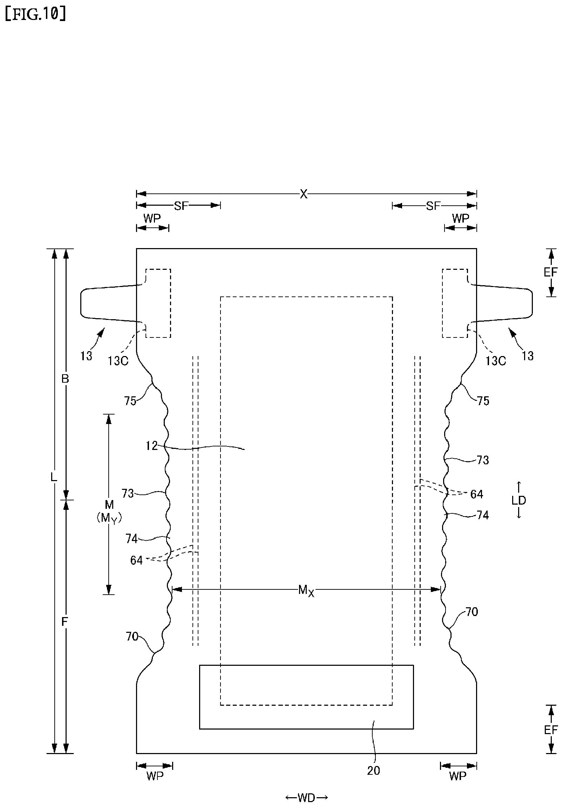

Both side edges 73 of the crotch portion M may be a wavy portion 80 similar to the lower edge 75 of the wing part WP in the range excluding at least both ends as in the example illustrated in Fig. 10. However, the crotch portion M is susceptible to leg movements. For this reason, if both side edges 73 of the crotch portion M are wavy portions 80, the projecting sections 74 may be folded between the surface of the diaper and the skin, which may lead to leakage and deterioration of wearing feeling. Therefore, unlike the lower edge 70 of the wing part WP, it is preferable that both side edges 73 of the crotch portion M extend linearly in a direction inclined obliquely inward by 1 to 2 degrees toward the front, as in the example illustrated in Fig. 2 (alternatively, if both the side edges 73 of the crotch portion pass through a width range of ± 5 mm away vertically from a direction intersecting with the front-back direction LD at the smaller angle θ₀ less than ± 2 degrees, a curved line without an inflection point is also acceptable). As described above, when the width of the crotch portion M becomes narrower toward the front, the thighs are easily move forward. Therefore, when the thigh is bent, the fitting between the upper front surface of the thigh and the lower edge 70 of the wing part of the ventral part F is improved.

The wing part WP having the first part 71 and the second part 72 as described above may be formed by a member different from the other portions. However, in the structure having the side flap portion SF as in the illustrated the example, it is preferable that the leg peripheral edge from the side edge of the crotch portion M to the lower edge 70 of the wing part is formed by cutting the middle of the side portion of the side flap portion SF in the front-back direction LD to make manufacturing easy.

In addition, as in the illustrated example, it is preferable that, on the width direction WD center side of the wing part WP of the ventral part F in the side flap portion SF, the side elastic member 64 is provided, and also a flat gather that is contracted in the front-back direction LD by this side elastic member 64 is provided, the front-back direction range in which contraction force of the side elastic member 64 acts (in the illustrated example, the range in the front-back direction LD of the portion having the side elastic member 64) has an overlapping range 77 overlapping at least a part (particularly preferably all) of the front-back direction range of the first part 71, and the first part 71 has a portion contracted by the side elastic member 64 in a natural length state. As a result, if a portion having the first part 71 can also be expanded and contracted to some extent in the front-back direction LD together with the flat gather. Therefore, in a wide dimensions around legs, the fitting between the upper front surface of the thigh and the lower edge 70 of the wing part of the ventral part F when the thigh is bent is improved. Unlike the illustrated example, by extending to a position where the side elastic member 64 overlaps at least a part (particularly preferably all) of the front-back direction LD range of the second part 72, the overlapping range 77 may extend over the entire wavy portion 80 of the first part 71.

In the case of having such the overlapping range 77, the maximum elongation of the flat gather is 150 to 350% (more preferably 200 to 300%), the width direction intervals 64i and 64j between the side elastic member 64 and the first part 71 in the overlapping range 77 preferably has the maximum interval 64j of 15 to 50 mm (more preferably 18 to 25 mm) and the minimum interval 64i of 5 to 20 mm (more preferably 10 to 17 mm). When the distance between the first part 71 and the side elastic member 64 and the maximum elongation of the flat gather are within the range, it is preferable that the first part 71 has a frill that is gently wavy in the thickness direction and has an excellent aesthetic appearance. In particular, when such a frill is combined with the shape of the first part 71, there is an advantage that the aesthetic appearance of the frill is further improved. On the other hand, if the distance between the first part 71 and the side elastic member 64 is excessively short, or if the maximum elongation of the flat gathers is excessively large, the finer frill waves not only reduce frill-likeness, but may also make the touch feeling harder. Further, if the distance between the first part 71 and the side elastic member 64 is excessively large, the projecting sections 74 may be folded between the surface of the diaper and the skin, which may also lead to leakage and deterioration of wearing feeling. On the other hand, if the distance between the first part 71 and the side elastic member 64 is excessively large, or if the maximum elongation of the flat gathers is excessively short, the frill formation may be insufficient. The maximum elongation means, as described above, the maximum value of elongation in a stretchable direction (in other words, elongation in a spread state when the flat gather expands flatly without contraction or slack), and the length in a spread state is expressed as a percentage when the natural length is 100%.

On the other hand, normally the thigh has a small angle to bend backward. The wing part WP of the dorsal part B preferably covers the gluteal region widely. Therefore, it is preferable that the lower edge 75 of the wing part WP of the dorsal part B greatly bend laterally from the back end of the crotch portion M (asymmetrical in the front and back direction), unlike the wing part WP of the ventral part F. Further, as with the wing part WP of the ventral part F, it is preferable that, in the lower edge 75 of the wing part WP of the dorsal part B, at least a range excluding both ends (preferably the whole as in the illustrated example) be the wavy portion 80 having a plurality of valleys recessed inward. Specifically, as illustrated in Figs. 7(b) and 8(a), it is preferable that in the wavy portion 80 at the lower edge 75 of the wing part WP of the dorsal part B, an obtuse side intersection angle θ₃ between the tangent D3 contacting both of the adjacent valleys 83 and the front-back direction be 120 to 150 degrees, the dimension 75y of the lower edge 75 of the wing part WP of the dorsal part B in the front-back direction LD be 0.2 to 0.3 times the dimension M_{Y} of the crotch portion M in the front-back direction LD.

Since the lower edge 75 of the wing part WP of the dorsal part B is also the wavy portion 80, there is the same advantage as the lower edge 70 of the wing part of the ventral part F. That is, also in the dorsal part B, the position of the lower edge 75 of the wing part can be changed according to the degree of bending deformation of the projecting sections 74 (the portions corresponding to the width of the wave). Then, since this projecting section 74 is deformed by contact with the body surface, the degree of bending deformation of the projecting section 74 is determined by the difference in body size and the degree of bending of the thigh. That is, the projecting section 74 is deformed according to the difference in body size, the degree of bending of the thigh, and the like, and fits the body surface. In particular, when the thigh is greatly bent, the projecting sections 74 are bent, and the friction and contact between the lower edge 75 and the thigh can be reduced. Moreover, rather than simply expanding the space under the wing parts WP, the projecting section 74 covers the largest possible area. For this reason, it is easy to fit regardless of a difference in body size and bending of the thigh, and anxiety about leakage from around the leg can be reduced. From these viewpoints, it is preferable that the lower edge 75 of the wing part WP of the dorsal part B has about two to five projecting sections 74. The dimensions of the wavy portion 80 in the lower edge 75 of the wing part WP of the dorsal part B can be appropriately determined, but in the normal case, it is preferable that the maximum value of the total amplitude 82 be 3 to 10 mm, and the wavelength 85 be 10 to 20 mm. In particular, if the total amplitude 82 of the wavy portion 80 is excessively large, the projecting sections 74 may be folded between the surface of the diaper and the skin, which may lead to leakage and deterioration of wearing feeling. Note that the lower edge 70 of the wing part WP of the dorsal part B may not be the wavy portion 80 but may have another known shape.

Further, as in the illustrated example, it is preferable that the front-back direction range in which the contraction force of the side elastic member 64 acts (the range in the front-back direction LD of the portion having the side elastic member 64 in the illustrated example) have the overlapping range 77 that overlaps at least a part (particularly preferably all) of the front-back direction range of the lower edge 75 in the wing part WP of the dorsal part B. The maximum elongation of the flat gather in this case and the width direction intervals 64i and 64j between the lower edge 75 of the wing part WP of the dorsal part B and the side elastic member 64 in the overlapping range 77 are the same as that in the case of the ventral part described above. The advantages of such a structure are basically the same as those of the ventral part F.

In the illustrated example, both the ventral part F and the dorsal part B have an overlapping range 77, but only one may have an overlapping range 77, and the other may not have an overlapping range 77. Alternatively, the overlapping ranges 77 may be provided, but the conditions such as having the intervals 64i and 64j described above may not be satisfied.

### <Explanation of Terms Used Herein>

The following terms in the specification have the following meanings unless otherwise specified in the specification.

"Front-back direction" means the direction indicated by the reference character LD in the drawings (longitudinal direction), and "width direction" means the direction indicated by WD in the drawings (left-right direction), and the front-back direction and the width direction are orthogonal to each other.

"Curve" means not including a straight line.

"Spread state" means a flatly spread state without shrinkage or slackness.

"Stretch rate" means the value when the natural length is taken as 100%. For example, a stretch rate of 200% is synonymous with a stretch magnification of 2 times.

"Gel strength" is measured as follows: A super absorbent polymer 1.0 g is added to artificial urine (urea: 2 wt%, sodium chloride: 0.8 wt%, calcium chloride dihydrate: 0.03 wt%, magnesium sulfate heptahydrate: 0.08 wt%, and ion exchanged water: 97.09 wt%) 49.0 g, and stirred with a stirrer. After leaving generated gel for three hours in a thermo-hygrostat bath at 40°C × 60%RH, return to room temperature, and measure the gel strength with a card meter (Curdmeter-MAX ME-500, manufactured by I. techno Engineering).

"Basis weight" is measured as follows. After preliminary drying a sample or a test piece, the sample or the test piece is left in a test chamber or equipment in the standard state (the test location is at a temperature of 23 ± 1°C and with a relative humidity of 50 ± 2%) to be constant weight. The preliminary drying refers to making a sample or a test piece a constant weight in an environment at a temperature of 100°C. For fibers with an official moisture regain of 0.0%, preliminary drying may not be performed. A sample of dimensions of 100 mm × 100 mm is cut using a template for sampling (100 mm × 100 mm) from a test piece in a constant weight. The basis weight is set by weighing the sample, multiplying by 100, and calculating the weight per one square meter.

"Thickness" is automatically measured under the conditions of a load of 0.098 N/cm² and a pressing area of 2 cm² using an automatic thickness measuring device (KES-G5 handy compression testing machine).

"Water absorption capacity" is measured according to JIS K7223-1996 "Test method for water absorption capacity of super absorbent polymer".

"Water absorption rate" is the "time to end point" when JIS K7224-1996 "Test method for water absorption rate of super absorbent resin" has been carried out using 2 g of super absorbent polymer and 50 g of physiological saline solution.

When environmental conditions in tests and measurements are not described, the tests and measurements shall be carried out in a test room or apparatus in a standard state (a temperature of 23 ± 1°C and a relative humidity of 50 ± 2% at the test location).

The dimension of each part means the dimension in the spread state, not the natural length state, unless otherwise stated.

### Industrial Applicability

The present invention is applicable to a tape-type disposable diaper as in the above example.

### Reference Signs List

- 11: liquid impervious sheet
- 12: outer nonwoven fabric
- 13: connecting tape
- 13A: connecting portion
- 13B: tape main unit section
- 13C: tape attachment part
- 20: target sheet
- 30: top sheet
- 40: intermediate sheet
- 50: absorbent element
- 56: absorber
- 58: package sheet
- 60: rising gather
- 62: gather sheet
- 63: elastic member
- 64: side elastic member
- 64i: minimum width direction interval
- 64j: maximum width direction interval
- 70, 75: lower edge
- 70y, 75y: dimension
- 71: first part
- 71y: dimension of the first part 71
- 72: second part
- 72y: dimension of the second part 72
- 73: side edge
- 74: projecting section
- 77: overlapping range
- 80: wavy portion
- 81: direction
- 82: amplitude
- 83: valley
- 85: wavelength
- B: dorsal part
- F: ventral part
- L: maximum length of the diaper
- LD: front-back direction
- M: crotch portion
- M_{X}: minimum width of the crotch portion M
- M_{Y}: dimension of the crotch portion M
- SF: side flap portion
- EF: end flap portion
- WD: width direction
- WP: wing part
- θ₁: intersection angle
- D1: tangent
- Θ₂: intersection angle
- D2: tangent
- Θ₃: intersection angle
- D3: tangent

## Claims

1. A tape-type disposable diaper, comprising:
a ventral part (F) extending forward from the center in a front-back direction (LD);
a dorsal part (B) extending backward from the center in the front-back direction (LD);
a crotch portion (M), both side edges of which extend from the middle in the front-back direction (LD) of the ventral part (F) to the middle in the front-back direction (LD) of the dorsal part (B), both the side edges of the crotch portion (M) passing through a width range of ± 5 mm away vertically from a direction intersecting with the front-back direction (LD) at a smaller angle less than ± 2 degrees;
an absorber (56) incorporated in a range including the crotch portion (M);
wing parts (WP) extending outward in the width direction (WD) compared with the crotch portion (M) in the ventral part (F) and the dorsal part (B); and
a connecting tape (13) for detachably connecting the ventral part (F) both sides of the dorsal part (B),
wherein a lower edge (70, 75) of the wing part (WP) of the ventral part (F) extends so as to be located obliquely outward toward the front side,
the lower edge (70, 75) of the wing part (WP) of the ventral part (F) has a first part (71) located on the base edge side of the wing part (WP) and a second part (72) located on the tip end side of the wing part (WP),
the range excluding at least both ends of the first part (71) and the range excluding at least both ends of the second part (72) are each a wavy portion (80) having a plurality of valleys (83) recessed inward,
an obtuse side intersection angle between a tangent contacting both of adjacent valleys (83) and the front-back direction (LD) in the wavy portion (80) of the first part (71) is 160 to 175 degrees,
an obtuse side intersection angle between a tangent contacting both of adjacent valleys (83) and the front-back direction (LD) in a wavy portion (80) of the second part (72) is 120 to 150 degrees,
the dimension (71y) of the first part (71) in the front-back direction (LD) is 0.15 to 0.30 times the dimension of the crotch portion (M) in the front-back direction (LD), and
the dimension (72y) of the second part (72) in the front-back direction (LD) is 0.10 to 0.25 times the dimension (M_{y}) of the crotch portion (M) in the front-back direction (LD).

2. The tape-type disposable diaper according to claim 1,
wherein the wavy portion (80) of the first part (71) and the wavy portion (80) of the second part (72) each have a maximum total amplitude (82) of 3 to 10 mm and a wavelength (85) of 10 to 20 mm.

3. The tape-type disposable diaper according to claim 1 or 2,
wherein both the side edges of the crotch portion (M) extend straight or in a curved shape having no inflection point in a direction inclined obliquely inward by 1 to 2 degrees toward the front.

4. The tape-type disposable diaper according to any one of claims 1 to 3, comprising:
side flap portions (SF) having no absorber (56) on both sides in the width direction (WD) from the ventral part (F) to the dorsal part (B);
a side elastic member (64) provided on the width direction (WD) center side of the wing part (WP) of the ventral part (F) in the side flap portions (SF); and
a flat gather that contracts in a front-back direction (LD) by the side elastic member (64),
wherein a front-back direction (LD) range in which contraction force of the side elastic member (64) acts has an overlapping range (77) overlapping with at least a part of the front-back direction (LD) range of the first part (71) overlap, and
the first part (71) has a portion contracted by the side elastic member (64) in a natural length state.

5. The tape-type disposable diaper according to claim 4,
wherein the maximum elongation of the flat gather is 150 to 350%, and in the overlapping range (77), width direction (WD) intervals between the side elastic member (64) and the first part (71) is 15 to 50 mm at the maximum and 5 to 20 mm at the minimum.

6. The tape-type disposable diaper according to any one of claims 1 to 5,
wherein the lower edge (70, 75) of the wing part (WP) of the dorsal part (B) extends so as to be located obliquely outward toward the back side from the back end of the side edge of the crotch portion (M),
at least a range excluding both ends in the lower edge (70, 75) of the wing part (WP) of the dorsal part (B) is a wavy portion (80) having a plurality of valleys (83) recessed inward,
an obtuse side intersection angle between a tangent contacting both adjacent valleys (83) and the front-back direction (LD) in the wavy portion (80) at the lower edge (70, 75) of the wing part (WP) of the dorsal part (B) is 120 to 150 degrees, and
the front-back (LD) dimension of the lower edge (70, 75) of the wing part (WP) of the dorsal part (B) is 0.2 to 0.3 times the front-back (LD) dimension of the crotch portion (M).

## Patentansprüche

1. Wegwerfwindel vom Bandtyp, umfassend:
einen Bauchteil (F), der sich in einer Vorne-nach-hinten-Richtung (LD) von der Mitte aus nach vorne erstreckt;
einen Rückenteil (B), der sich in einer Vorne-nach-hinten-Richtung (LD) von der Mitte aus nach hinten erstreckt;
einen Schrittabschnitt (M), dessen beide Seitenränder sich von der Mitte in der Vorne-nach-hinten-Richtung (LD) des Bauchteils (F) zur Mitte in der Vorne-nach-hinten-Richtung (LD) des Rückenteils (B) erstrecken, wobei die Seitenränder des Schrittabschnitts (M) durch einen Breitenbereich von ± 5 mm vertikal weg von einer Richtung verlaufen, die sich mit der Vorne-nach-hinten-Richtung (LD) in einem kleineren Winkel von weniger als ± 2 Grad schneidet;
einen Absorber (56), der in einem Bereich eingefügt ist, der den Schrittabschnitt (M) einschließt;
Flügelteile (WP), die sich verglichen mit dem Schrittabschnitt (M) im Bauchteil (F) und im Rückenteil (B) in der Breitenrichtung (WD) nach außen erstrecken; und
ein Verbindungsband (13) zur lösbaren Verbindung des Bauchteils (F) mit beiden Seiten des Rückenteils (B),
wobei sich ein unterer Rand (70, 75) des Flügelteils (WP) des Bauchteils (F) so erstreckt, dass er sich schräg nach außen zur Vorderseite hin befindet,
wobei der untere Rand (70, 75) des Flügelteils (WP) des Bauchteils (F) einen ersten Teil (71), der sich auf der Seite des Basisrandes des Flügelteils (WP) befindet, und einen zweiten Teil (72), der sich auf der Seite des Spitzenendes des Flügelteils (WP) befindet, aufweist,
wobei der Bereich, der mindestens beide Enden des ersten Teils (71) ausschließt, und der Bereich, der mindestens beide Enden des zweiten Teils (72) ausschließt, jeweils ein wellenförmiger Abschnitt (80) mit einer Vielzahl von nach innen vertieften Tälern (83) sind,
ein stumpfer Seitenschnittwinkel zwischen einer Tangente, die beide benachbarte Täler (83) berührt, und der Vorne-nach-hinten-Richtung (LD) in dem welligen Abschnitt (80) des ersten Teils (71) 160 bis 175 Grad beträgt,
ein stumpfer Seitenschnittwinkel zwischen einer Tangente, die beide benachbarte Täler (83) berührt, und der Vorne-nach-hinten-Richtung (LD) in einem welligen Abschnitt (80) des zweiten Teils (72) 120 bis 150 Grad beträgt,
die Abmessung (71y) des ersten Teils (71) in der Vorne-nach-hinten-Richtung (LD) das 0,15- bis 0,30 -Fache der Abmessung des Schrittabschnitts (M) in der Vorne-nach-hinten-Richtung (LD) beträgt, und
die Abmessung (72y) des zweiten Teils (72) in der Vorne-nach-hinten-Richtung (LD) das 0,10- bis 0,25-Fache der Abmessung (M_{y}) des Schrittabschnitts (M) in der Vorne-nach-hinten-Richtung (LD) beträgt.

2. Wegwerfwindel vom Bandtyp nach Anspruch 1,
wobei der wellenförmige Abschnitt (80) des ersten Teils (71) und der wellenförmige Abschnitt (80) des zweiten Teils (72) jeweils eine maximale Gesamtamplitude (82) von 3 bis 10 mm und eine Wellenlänge (85) von 10 bis 20 mm aufweisen.

3. Wegwerfwindel vom Bandtyp nach Anspruch 1 oder 2,
wobei die beiden Seitenränder des Schrittabschnitts (M) sich gerade oder in einer gekrümmten Form ohne Knickpunkt in eine Richtung erstrecken, die um 1 bis 2 Grad schräg nach innen zur Vorderseite geneigt ist.

4. Wegwerfwindel vom Bandtyp nach einem der Ansprüche 1 bis 3, umfassend:
Seitenklappenabschnitte (SF) ohne Absorber (56) auf beiden Seiten in der Breitenrichtung (WD) vom Bauchteil (F) zum Rückenteil (B);
ein seitliches elastisches Element (64), das auf der mittleren Seite in Breitenrichtung (WD) des Flügelteils (WP) des Bauchteils (F) in den Seitenklappenabschnitten (SF) vorgesehen ist; und
eine flache Raffung, die sich durch das seitliche elastische Element (64) in einer Vorne-nach-hinten-Richtung (LD) zusammenzieht,
wobei ein Bereich der Vorne-nach-hinten-Richtung (LD), in dem die zusammenziehende Kraft des seitlichen elastischen Elements (64) wirkt, einen Überlappungsbereich (77) aufweist, der mit mindestens einem Teil des Bereichs der Vorne-nach-hinten-Richtung (LD) des ersten Teils (71) überlappt, und
der erste Teil (71) einen Abschnitt aufweist, der durch das elastische Seitenelement (64) in einen Zustand natürlicher Länge zusammengezogen ist.

5. Wegwerfwindel vom Bandtyp nach Anspruch 4,
wobei die maximale Dehnung der flachen Raffung 150 bis 350 % beträgt und im Überlappungsbereich (77) die Abstände in Breitenrichtung (WD) zwischen dem elastischen Seitenelement (64) und dem ersten Teil (71) höchstens 15 bis 50 mm und mindestens 5 bis 20 mm betragen.

6. Wegwerfwindel vom Bandtyp nach einem der Ansprüche 1 bis 5,
wobei sich der untere Rand (70, 75) des Flügelteils (WP) des Rückenteils (B) so erstreckt, dass er sich vom hinteren Ende des Seitenrandes des Schrittabschnitts (M) schräg nach außen in Richtung der Rückseite erstreckt,
mindestens ein Bereich, der beide Enden am unteren Rand (70, 75) des Flügelteils (WP) des Rückenteils (B) ausschließt, ein wellenförmiger Abschnitt (80) mit einer Vielzahl von nach innen vertieften Tälern (83) ist,
ein stumpfer Seitenschnittwinkel zwischen einer Tangente, die beide benachbarten Täler (83) berührt, und der Vorne-nach-hinten-Richtung (LD) in dem welligen Abschnitt (80) an den unteren Rändern (70, 75) des Flügelteils (WP) des Rückenteils (B) 120 bis 150 Grad beträgt, und
die Vorne-nach-hinten-Abmessung (LD) des unteren Randes (70, 75) des Flügelteils (WP) des Rückenteils (B) das 0,2- bis 0,3 -Fache der Vorne-nach-hinten-Abmessung (LD) des Schrittabschnitts (M) beträgt.

## Revendications

1. Couche jetable de type à bande, comprenant :
une partie ventrale (F) s'étendant vers l'avant depuis le centre dans une direction avant/arrière (LD) ;
une partie dorsale (B) s'étendant vers l'arrière depuis le centre dans la direction avant/arrière (LD) ;
une portion d'entrejambe (M), dont les deux bords latéraux s'étendent depuis le milieu dans la direction avant/arrière (LD) de la partie ventrale (F) jusqu'au milieu dans la direction avant/arrière (LD) de la partie dorsale (B), les deux bords latéraux de la portion d'entrejambe (M) passant par une zone de largeur de ±5 mm en éloignement verticalement d'une direction recoupant la direction avant/arrière (LD) sous un nombre plus petit inférieur à ±2 degrés ;
un absorbant (56) incorporé dans une zone incluant la portion d'entrejambe (M) ; des parties en forme d'ailes (WP) s'étendant vers l'extérieur dans la direction de la largeur (WD) par comparaison à la portion d'entrejambe (M) dans la partie ventrale (F) et la partie dorsale (B) ; et
une bande de connexion (13) pour connecter de manière détachable la partie ventrale (F) des deux côtés de la partie dorsale (B),
dans laquelle un bord inférieur (70, 75) de la partie en forme d'aile (WP) de la partie ventrale (F) s'étend de manière à être situé en oblique vers l'extérieur en direction de l'avant,
le bord inférieur (70, 75) de la partie en forme d'aile (WP) de la partie ventrale (F) a une première partie (71) située sur le côté du bord de base de la partie en forme d'aile (WP) et une seconde partie (72) située sur le côté d'extrémité de pointe de la partie en forme d'aile (WP),
la zone excluant au moins les deux extrémités de la première partie (71) et la zone excluant au moins les deux extrémités de la seconde partie (72) sont chacune une portion ondulée (80) ayant une pluralité de vallées (83) en évidement vers l'intérieur,
un angle d'intersection latéral obtus entre une tangente contactant les deux vallées adjacentes (83) et la direction avant/arrière (LD) dans la portion ondulée (80) de la première partie (71) est de 160 à 175 degrés,
un angle d'intersection latéral obtus entre une tangente contactant les deux vallées adjacentes (83) et la direction avant/arrière (LD) dans la portion ondulée (80) de la seconde partie (72) est de 120 à 150 degrés,
la dimension (71y) de la première partie (71) dans la direction avant/arrière (LD) est de 0,15 à 0,30 fois la dimension de la portion d'entrejambe (M) dans la direction avant/arrière (LD), et
la dimension (72y) de la seconde partie (72) dans la direction avant/arrière (LD) est de 0,10 à 0,25 fois la dimension (My) de la portion d'entrejambe (M) dans la direction avant/arrière (LD).

2. Couche jetable de type à bande selon la revendication 1,
dans laquelle la portion ondulée (80) de la première partie (71) et la portion ondulée (80) de la seconde partie (72) ont chacune une amplitude totale maximum (82) de 3 à 10 mm et une longueur d'onde (85) de 10 à 20 mm.

3. Couche jetable de type à bande selon la revendication 1 ou 2,
dans laquelle les deux bords latéraux de la portion d'entrejambe (M) s'étendent en ligne droite ou dans une forme incurvée n'ayant aucun point d'inflexion dans une direction inclinée en oblique vers l'intérieur à raison de 1 à 2 degrés vers l'avant.

4. Couche jetable de type à bande selon l'une quelconque des revendications 1 à 3, comprenant :
des portions de rabat latérales (SF) n'ayant aucun absorbant (56) sur les deux côtés dans la direction de la largeur (WD) depuis la partie ventrale (F) jusqu'à la partie dorsale (B) ;
un élément élastique latéral (64) prévu sur le côté central dans la direction de la largeur (WD) de la partie en forme d'aile (WP) de la partie ventrale (F) dans les portions de rabat latérales (SF) ; et
une fronce plate qui se contracte dans une direction avant/arrière (LD) via l'élément élastique latéral (64),
dans laquelle une zone dans la direction avant/arrière (LD) dans laquelle une force de contraction de l'élément élastique latéral (64) agit a une zone de chevauchement (77) qui chevauche au moins une partie de la zone dans la direction avant/arrière (LD) du chevauchement de la première partie (71), et
la première partie (71) a une portion contractée par l'élément élastique latéral (64) dans un état de longueur naturelle.

5. Couche jetable de type à bande selon la revendication 4,
dans laquelle l'allongement maximum de la fronce plate est de 150 à 350 %, et dans la plage de chevauchement (77), des intervalles dans la direction de la largeur (WD) entre l'élément élastique latéral (64) et la première partie (71) sont de 15 à 50 mm au maximum est de 50 à 20 mm au minimum.

6. Couche jetable de type à bande selon l'une quelconque des revendications 1 à 5,
dans laquelle le bord inférieur (70, 75) de la partie en forme d'aile (WP) de la partie dorsale (B) s'étend de manière à être situé en oblique vers l'extérieur vers le côté arrière depuis l'extrémité arrière du bord latéral de la portion d'entrejambe (M),
au moins une zone excluant les deux extrémités dans le bord inférieur (70, 75) de la partie en forme d'aile (WP) de la partie dorsale (B) est une portion ondulée (80) ayant une pluralité de vallées (83) en évidement vers l'intérieur,
un angle d'intersection latéral obtus entre une tangente contactant les deux vallées adjacentes (83) et la direction avant/arrière (LD) dans la portion ondulée (80) au niveau du bord inférieur (70, 75) de la partie en forme d'aile (WP) de la partie dorsale (B) est de 120 à 150 degrés, et
la dimension avant/arrière (LD) du bord inférieur (70, 75) de la partie en forme d'aile (WP) de la partie dorsale (B) est de 0,2 à 0,3 fois la dimension avant/arrière (LD) de la portion d'entrejambe (M).
